# EUROPÄISCHE PATENTSCHRIFT

(11) **EP 0 267 594 B1**
(45) Veröffentlichungstag und Bekanntmachung des Hinweises auf die Patenterteilung: **10.02.1993**
(21) Anmeldenummer: 87116630.2
(22) Anmeldetag: 11.11.1987
(51) Int. Cl.: C07D 239/50, C07B 43/04

(54) **Verbessertes Verfahren zur katalytischen Hydrierung von DAHNP und zur Herstellung von DAFHP**
Method for the catalytic hydrogenation of DAHNP and for preparing DAFHP
Méthode pour l'hydrogénation catalytique de DAHNP et pour la préparation de DAFHP

(30) Priorität: 12.11.1986 DE 3638635
(43) Veröffentlichungstag der Anmeldung: 18.05.1988
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Schneider, Heinrich, Dr., D-6507 Ingelheim am Rhein (DE)

(56) Entgegenhaltungen:
- US-A- 2 447 523
- Chemical Abstracts, Band 105, Nr. 4, 28. Juli 1986, Columbus, Ohio, US Yoshida, Kokichi; Sueoka, Motoyuki, S. 515, Spalte 1, Zusammenfassung-Nr. 31930u
- Chemical Abstracts, Band 105, Nr. 10, 8. September 1986, Columbus, Ohio, US Yoshida, Kokichi; Sueoka, Motoyuki, S. 574, Spalte 2, Zusammenfassung-Nr. 87511a

## Beschreibung

Die Erfindung betrifft ein verbessertes Verfahren zur katalytischen Hydrierung von 2,4-Diamino-6-hydroxy-5-nitrosopyrimidin (DAHNP) und die Umsetzung des dabei erhaltenen 2,4,5-Triamino-6-hydroxy-pyrimidin (TAHP) mit Ameisensäure zu 2,4-Diamino-5-formylamino-6-hydroxy-pyrimidin (DAFHP).

Die katalytische Hydrierung von DAHNP ist bekannt und wird beispielsweise in der US-Patentschrift 2 447 523 beschrieben. Die Verfahrensparameter werden dort wie folgt angegeben. Eine 0.4- bis 0.5 molare Suspension von DAHNP in 0.1 bis 1 normaler Natronlauge (entsprechend 0,2 bis 2,5 mol NaOH pro mol Nitrosoverbindung) wird unter Verwendung von Pd, PtO₂ oder Raney-Nickel als Katalysator bei Wasserstoffdrücken von 1,4 bis 2,3 bar hydriert.

Im Rahmen einer technisch durchgeführten Synthese ist es nicht nur von Interesse, eine Reaktion mit möglichst hohen Ausbeuten durchzuführen, sondern auch einen möglichst hohen Durchsatz (gemessen in kg/h), bzw. eine hohe Raum-Zeit-Ausbeute (kg/h 1) zu erreichen. Dies gilt insbesondere für Reaktionen, bei denen das Reaktorvolumen, wie z.B. bei Reaktionen in Autoklaven, nicht ohne größere Aufwendungen geändert werden kann.

Bei vorgegebenen Reaktorvolumen ist der Durchsatz des in der US-PS beschriebenen Verfahrens durch die in der Patentschrift angegebenen Konzentrationen an DAHNP von 0,4 bis 0,5 mol/l begrenzt. Eine merkliche Erhöhung der Konzentrationen an Ausgangsmaterial ist insofern nicht möglich, da sich dabei das Reaktionsgemisch derart verdickt, daß es praktisch nicht mehr gerührt werden kann und infolgedessen der Stoffübergang des Wasserstoffs in die flüssige Phase nahezu zum Erliegen kommt. Weder eine Erhöhung der Reaktionstemperatur, noch ein erhöhter wasserstoffdruck bringen in dieser Hinsicht eine Besserung.

Es ist die Aufgabe der vorliegenden Erfindung, ein Verfahren zur Hydrierung von DAHNP bereitzustellen, das bei gleichem Reaktorvolumen einen größeren Durchsatz ermöglicht.

Erfindungsgemäß wird die Aufgabe dadurch gelöst, daß eine wässerige, bis drei molare Suspension von DAHNP in Gegenwart eines geeigneten Katalysators hydriert wird, wobei eine Base kontinuierlich während der gesamten Hydrierdauer zudosiert wird. Im allgemeinen erfolgt die Umsetzung bei Wasserstoffdrücken von 1,4 bis 21 bar und bei Temperaturen zwischen 20 und 80°C.

Die Konzentration an DAHNP beträgt 0,5 bis 3 mol/l, bevorzugt 2 bis 2,5 mol/l. Vorzugsweise wird die Hydrierung bei wasserstoffdrücken zwischen 2 und 5 bar durchgeführt. Geeignete Hydrierkatalysatoren sind dem Fachmann hinreichend bekannt, bevorzugt werden PtO₂, Pd oder Raney-Nickel, besonders bevorzugt ist Pd-Kohle mit einem Pd-Gehalt zwischen 1 und 10 %, bevorzugt 2,5 %, in einer Konzentration von 0,02 bis 0,2 g, vorzugsweise 0,1 g, Palladium pro Mol Nitrosoverbindung. Der bevorzugte Temperaturbereich liegt zwischen 40 und 60°C.

Die Menge an zugesetzter Base ist in weiten Bereichen unkritisch, wobei im allgemeinen 0,8 bis 1,5 Mol Base pro Mol eingesetztes DAHNP, vorzugsweise 1,1 Mol, geeigneterweise in Form einer wässerigen Lösung, Verwendung findet. Als Basen sind Alkalihydroxide, wie z.B. NaOH oder KOH bevorzugt. Eine andere geeignete Base ist beispielsweise Ammoniumhydroxid. Die Base wird beispielsweise in Form einer 50 %igen wässerigen Lösung zu dem Reaktionsgemisch zugegeben, wobei die Dosierung zweckmäßigerweise kontinuierlich, proportional zum Umsatz erfolgt. Die Reaktion kann in allen herkömmlichen Druckreaktoren mit Rühr- oder Mischeinrichtungen durchgeführt werden, bevorzugt ist jedoch die Durchführung in einem Treibstrahl-Schlaufenreaktor.

Die freie Base des bei der Reduktion entstehenden 2,4,5-Triamino-6-hydroxypyrimidins ist oxidationsempfindlich und neigt an der Luft zu Verfärbungen. Wie bekannt, kann das TAHP durch Zugabe von Schwefelsäure als stabiles Sulfat-Hydrat in nahezu quantitativer Ausbeute isoliert werden.

Der Vorteil des erfindungsgemäßen Verfahrens liegt darin, daß die Raum-Zeit-Ausbeute (kg/h 1) bei gleichem Reaktortyp nahezu versechsfacht ist, wobei jedoch nur nahezu gleiche Mengen an Katalysator und Base aufgewendet werden müssen.

Ein weiterer Vorteil des erfindungsgemäßen Hydrierverfahrens ergibt sich bei der nachfolgenden Umsetzung des entstandenen Reaktionsproduktes zu DAFHP. Nach Abfiltrieren des Katalystors direkt aus der Hydrierlösung wird durch Umsetzung mit Ameisensäure, gegebenenfalls unter Zusatz einer Mineralsäure, in nahezu quantitativer Ausbeute das 2,4-Diamino-5-formylamino-6-hydroxy-pyrimidin (DAFHP) erhalten. Anstelle von Mineralsäure kann auch eine erhöhrte Menge Ameisensäure eingesetzt werden. Dies bietet zweierlei Vorteile. Zum einen können auf diese Weise Apparate und Gerätschaften Verwendung finden, die nicht aus mineralsäurebeständigen Materialien bestehen, zum anderen unterbleibt eine Belastung des Abwassers mit anorganischen Salzen. Im allgemeinen werden 1 bis 3,5 Mol, vorzugsweise bis 2,5 Mol, besonders bevorzugt 2 - 2,2 Mol Ameisensäure und 0,8 bis 1,5 Mol Mineralsäure, wie z.B. HCl oder HBr, pro Mol DAFHP verwandt; anstelle der Mineralsäure, können auch insgesamt 2 bis 5 Mol, bevorzugt 3,3 bis 4 Mol, Ameisensäure pro Mol DAFHP eingesetzt werden.

Die Umsetzung erfolgt beispielweise durch Vorlegen der entsprechenden Säureäquivalente und Zugabe der filtrierten Hydrierlösung, hierbei erwärmt sich das Reaktionsgemisch auf ca. 80 - 90°C. Die Umsetzung zur Formylverbindung wird durch weiteres Erwärmen auf 80° - 90°C vervollständigt. Nach dem Abkühlen fällt das DAFHP aus und wird nach bekannten Methoden isoliert.

Aus gering konzentrierten Lösungen, wie sie beispielsweise nach dem Verfahren der US-PS 2 447 523 erhalten werden, kann das DAFHP lediglich mit verminderter Ausbeute isoliert werden.

Die folgenden Beispiele sollen die Erfindung erläutern, ohne sie jedoch einzuschränken.

### Beispiel 1

In einem Rührautoklaven werden 279 g 2,4-Diamino-6-hydroxy-5-nitrosopyrimidin, 10 g 2,5 %-ige Pd-Kohle und 650 ml Wasser vorgelegt, auf 40°C erwärmt und mit 3 bar Wasserstoff beaufschlagt. Während ca. 30 min werden bei max. 50°C kontinuierlich 158,4 g 50%ige Natronlauge zudosiert. Das Reaktionsgemisch wird dann über einen Druckfilter in 312,4 g gut gekühlte 50%-ige Schwefelsäure eingerührt. Nach Abkühlen auf 10°C wird abgesaugt, mit 2 x 450 ml Eiswasser gewaschen und im Vakuumtrockenschrank unter einem schwachen Stickstoffatom bei 40°C getrocknet.
- Ausbeute:: 450,1 g leicht violettstichige Kristalle von 2,4,5-Triamino-6-hydroxypyrimidin-Sulfat, Hydrat entspr. 97,3 % Ausbeute

### Beispiel 2

In einem Rührautoklaven werden 279 g 2,4-Diamino-6-hydroxy-5-nitroso-pyrimidin, 10 g 2,5%-ige Pd-Kohle und 650 ml Wasser vorgelegt, auf 40°C erhitzt und mit 3 bar Wasserstoff beaufschlagt. Während ca 30 min werden bei max 50°C kontinuierlich 134,6 g 25%-ige Ammoniaklösung zudosiert. Die weitere Behandlung erfolgt wie in Beispiel 1 beschrieben.
- Ausbeute:: 448,3 g entspr. 96,9% d.Th.

### Beispiel 3

In einem Rührautoklaven werden 279 g 2,4-Diamino-6-hydroxy-5-nitroso-pyrimidin, 10 g 2,5%-ige Pd-Kohle und 650 ml Wasser vorgelegt und mit 1,4 bar Wasserstoff beaufschlagt. Während ca. 2 h werden bei 20 - 25°C kontinuierlich 158,4 g 50%-ige Natronlauge zudosiert. Die weitere Behandlung erfolgt wie in Beispiel 1 beschrieben.
- Ausbeute:: 449,2 g entspr. 97,1% d.Th.

### Beispiel 4

In einem Rührautoklaven werden 279 g 2,4-Diamino-6-hydroxy-5-nitroso-pyrimidin, 5 g 1%-ige Pd-Kohle und 650 ml Wasser vorgelegt, auf 70°C erhitzt und mit 21 bar Wasserstoff beaufschlagt. Während ca. 30 min werden bei max. 80°C kontinuierlich 158,4 g 50%-ige Natronlauge zudosiert. Die weitere Behandlung erfolgt wie in Beispiel 1 beschrieben.
- Ausbeute:: 446.9 g entspr. 96,6% d.Th.

### Beispiel 5

In einem VA-Treibstrahl-Schlaufenreaktor werden 46,5 kg 2,4-Diamino-6-hydroxy-5-nitrosopyrimidin, 1,5 kg 2,5%ige Pd-Kohle und 57,6 l Wasser eingezogen, auf 40°C erwärmt und mit 5 bar Wasserstoff beaufschlagt. Während ca. 30 min werden bei bis zu 50°C 26,5 kg 50 %-ige Natronlauge kontinuierlich zudosiert. Das Reaktionsgemisch wird dann über ein Linsenfilter in einen gut gekühlten 250 l-Email-Apparat mit 104,1 kg 50 %-iger Schwefelsäure unter Rühren eingebracht. Nach Abkühlen auf 10°C wird abgeschleudert, mit 2 x 75 l Wasser gewaschen und im Vakuumtrockenschrank bei 40°C getrocknet.
Ausbeute 75,5 kg (97,9 %) 2,4,5-Triamino-6-hydroxypyrimidin-Sulfat, Hydrat

### Beispiel 6

Nach der Hydrierung wie in Beispiel 1 beschrieben, wird das Reaktionsgemisch über ein Druckfilter in 182,2 g Ameisensäure eingerührt. Nach Zugabe von 254,6 g 63 %iger Bromwasserstoffsäure wird 2 h auf 85°C erhitzt. Man kühlt auf 10°C ab, saugt ab und wäscht mit 3 x 300 ml Wasser. Nach Trocknen im Vakuumtrockenschrank bei 60°C werden 296,0 g
2,4-Diamino-5-formylamino-6-hydroxypyrimidin (97,3 % Ausbeute) erhalten.

### Beispiel 7

Nach der Hydrierung wie in Beispiel 1 beschrieben, wird das Reaktionsgemisch über ein Druckfilter in 273,2 g Ameisensäure eingerührt. Man erhitzt noch 2 h auf 85°C, kühlt auf 10°C ab, saugt ab und wäscht mit 2 x 300 ml Wasser. Nach Trocknen im Vakuumtrockenschrank bei 60°C werden 296,9 g
2,4-Diamino-5-formylamino-6-hydroxy- pyrimidin (97,6 % Ausbeute) erhalten.

## Patentansprüche

1. Verfahren zur katalytischen Hydrierung von 2,4-Diamino-6-hydroxy-5-nitrosopyrimidin (DAHNP) zur Herstellung von 2,4,5-Triamino-6- hydroxypyrimidin (TAHP) in wäßriger Lösung, dadurch gekennzeichnet, daß die Konzentration an DAHNP 0,5 bis 3 mol/l beträgt und insgesamt 0,8 bis 1,5 Mol Base pro Mol DAHNP kontinuierlich während der gesamten Reaktionszeit zugesetzt werden.

2. Verfahren nach Anspruch 1, dadurch gekennzeichnet, daß als Base eine wässerige Lösung von KOH oder NaOH verwendet wird.

3. Verfahren nach Anspruch 1 oder 2, dadurch gekennzeichnet, daß die Hydrierung bei Wasserstoffdrücken zwischen 1,4 und 21 bar durchgeführt wird.

4. Verfahren nach einem der Ansprüche 1 bis 3, dadurch gekennzeichnet, daß die Hydrierung bei Temperaturen zwischen 20 und 80°C durchgeführt wird.

5. Verfahren nach einem der Ansprüche 1 bis 4, dadurch gekennzeichnet, daß als Hydrierkatalysator Pd, Pd/C, PtO₂ oder Raney-Nickel verwendet wird.

6. Verfahren nach einem der Ansprüche 1 bis 5, dadurch gekennzeichnet, daß die katalytische Hydrierung in einem Treibstrahl-Schlaufenreaktor durchgeführt wird.

7. Verfahren gemäß Anspruch 1, dadurch gekennzeichnet, daß eine alkalische Lösung des erhaltenen 2,4,5-Triamino-6-hydroxypyrimidins mit 1 bis 3,5 mol Ameisensäure und 0,8 bis 1,5 mol Mineralsäuren in 2,4-Diamino-5-formylamino-6-hydroxypyrimidin umgesetzt wird.

8. Verfahren gemäß Anspruch 1, dadurch gekennzeichnet, daß eine alkalische Lösung des erhaltenen 2,4,5-Triamino-6-hydroxypyrimidins mit 2 bis 5 mol Ameisensäure in 2,4-Diamino-5-formylamino-6-hydroxypyridinin umgesetzt wird.

9. Verfahren gemäß Anspruch 1, dadurch gekennzeichnet, daß eine alkalische Lösung des erhaltenen 2,4,5-Triamino-6-hydroxypyrimidins mit Ameisensäure, gegebenenfalls unter Zusatz von Mineralsäuren in 2,4-Diamino-5-formylamino-6--hydroxypyridin umgesetzt wird.

## Claims

1. Process for the catalytic hydrogenation of 2,4-diamino-6-hydroxy-5-nitrosopyrimidine (DAHNP) for the preparation of 2,4,5-triamino-6-hydroxypyrimidine (TAHP) in aqueous solution, characterised in that the concentration of DAHNP is 0.5 to 3 mol/l and a total of 0.8 to 1.5 mol of base are added continously, per mol of DAHNP, throughout the entire reaction time.

2. Process as claimed in claim 1, characterised in that an aqueous solution of KOH or NaOH is used as base.

3. Process as claimed in claim 1 or 2, characterised in that the hydrogenation is carried out at hydrogen pressures of between 1.4 and 21 bar.

4. Process as claimed in one of claims 1 to 3, characterised in that the hydrogenation is carried out at temperatures of between 20 and 80°C.

5. Process as claimed in one of claims 1 to 4, characterised in that Pd, Pd/c, PtO₂ or Raney nickel is used as the hydrogenation catalyst.

6. Process as claimed in one of claims 1 to 5, characterised in that the catalytic hydrogenation is carried out in a power jet-type loop reactor.

7. Process according to claim 1, characterised in that an alkaline solution of the 2,4,5-triamino-6-hydroxypyrimidine obtained is reacted with 1 to 3.5 mol of formic acid and 0.8 to 1.5 mol of inorganic acids to obtain 2,4-diamino-5-formylamino-6-hydroxypyrimidine.

8. Process according to claim 1, characterised in that an alkaline solution of the 2,4,5-triamino-6-hydroxypyrimidine obtained is reacted with 2 to 5 mol of formic acid to obtain 2,4-diamino-5-formylamino-6-hydroxypyrimidine.

9. Process according to claim 1, characterised in that an alkaline solution of the 2,4,5-triamino-6-hydroxypyrimidine obtained is reacted with formic acid, optionally with the addition of inorganic acids, to obtain 2,4-diamino-5-formylamino-6-hydroxypyrimidine.

## Revendications

1. Procédé d'hydrogénation catalytique de la 2,4-diamino-6-hydroxy-5-nitrosopyrimidine (DAHNP) pour la préparation de la 2,4,5-triamino-6-hydroxypyrimidine (TAHP) en solution aqueuse, caractérisé en ce que la concentration en DAHNP est de 0,5 à 3 mol/l et en ce que l'on ajoute en continu au total 0,8 à 1,5 mole de base par mole de DAHNP pendant toute la durée de la réaction.

2. Procédé selon la revendication 1, caractérisé en ce que l'on utilise comme base une solution aqueuse de KOH ou de NaOH.

3. Procédé selon la revendication 1 ou 2, caractérisé en ce que l'hydrogénation est réalisée à des pressions d'hydrogène comprises entre 1,4 et 21 bar.

4. Procédé selon l'une des revendications 1 à 3, caractérisé en ce que l'hydrogénation est réalisée à des températures comprises entre 20 et 80°C.

5. Procédé selon l'une des revendications 1 à 4, caractérisé en ce que l'on utilise comme catalyseur d'hydrogénation Pd, Pd/C, PtO₂ ou le nickel de Raney.

6. Procédé selon l'une des revendications 1 à 5, caractérisé en ce que l'hydrogénation catalytique est réalisée dans une colonne à bulles à écoulement en boucles et à jet d'entraînement.

7. Procédé selon la revendication 1, caractérisé en ce qu'une solution alcaline de la 2,4,5-triamino-6-hydroxypyrimidine obtenue est convertie en 2,4-diamino-5-formylamino-6-hydroxypyrimidine avec 1 à 3,5 moles d'acide formique et 0,8 à 1,5 mole d'acides minéraux.

8. Procédé selon la revendication 1, caractérisé en ce qu'une solution alcaline de la 2,4,5-triamino-6-hydroxypyrimidine obtenue est convertie en 2,4-diamino-5-formylamino-6-hydroxyprimidine avec 2 à 5 moles d'acide formique.

9. Procédé selon la revendication 1, caractérisé en ce qu'une solution alcaline de la 2,4,5-triamino-6-hydroxypyrimidine obtenue est convertie en 2,4-diamino-5-formylamino-6-hydroxypyrimidine avec l'acide formique, éventuellement avec addition d'acides minéraux.
